# EUROPÄISCHE PATENTSCHRIFT

(11) **EP 3 818 367 B1**
(45) Veröffentlichungstag und Bekanntmachung des Hinweises auf die Patenterteilung: **22.11.2023**
(21) Anmeldenummer: 19735245.3
(22) Anmeldetag: 26.06.2019
(51) Int. Cl.: G01N 27/417, G01N 33/00

(54) **VERFAHREN ZUM BETREIBEN EINES SENSORS ZUM NACHWEIS MINDESTENS EINES ANTEILS EINER MESSGASKOMPONENTE MIT GEBUNDENEM SAUERSTOFF IN EINEM MESSGAS**
METHOD FOR OPERATING A SENSOR FOR DETECTING AT LEAST ONE PORTION OF A MEASUREMENT GAS COMPONENT HAVING BOUND OXYGEN IN A MEASUREMENT GAS
PROCÉDÉ POUR FAIRE FONCTIONNER UN CAPTEUR DESTINÉ À DÉTECTER AU MOINS UNE FRACTION D'UN COMPOSANT D'UN GAZ DE MESURE AVEC DE L'OXYGÈNE LIÉ DANS UN GAZ DE MESURE

(30) Priorität: 03.07.2018 DE 102018210876
(43) Veröffentlichungstag der Anmeldung: 12.05.2021
(73) Patentinhaber: Robert Bosch GmbH, 70442 Stuttgart (DE)
(72) Erfinder: OECHTERING, Peter, 76229 Karlsruhe (DE); HEISE, Michael, 01445 Radebeul (DE); GUEL, Mustafa, 71522 Backnang (DE); FIEDLER, Michael, 71034 Boeblingen (DE); SCHROEDER, Andy, 5405 Daettwil (CH); SINGER, Matthias, 73730 Esslingen (DE); DAECKE, Dirk, 70619 Stuttgart (DE)
(86) Internationale Anmeldenummer: PCT/EP2019/066944
(87) Internationale Veröffentlichungsnummer: WO 2020/007674

(56) Entgegenhaltungen:
- DE-A1-102014 224 943
- DE-A1-102015 210 473
- DE-A1-102016 209 924
- US-A1- 2004 238 378
- US-A1- 2007 119 708

## Beschreibung

### Stand der Technik

Aus dem Stand der Technik ist eine Vielzahl von Verfahren und Sensoren zum Nachweis mindestens eines Anteils der Messgaskomponente mit gebundenem Sauerstoff in einem Gasgemisch, insbesondere in einem Abgas einer Verbrennungskraftmaschine, durch Erfassen eines Anteils an Sauerstoff, der durch eine Reduktion der Messgaskomponente mit dem gebundenen Sauerstoff erzeugt wird, bekannt.

Sensoren zum Nachweis mindestens eines Anteils der Messgaskomponente mit gebundenem Sauerstoff in einem Gasgemisch, die auch verkürzt oder vereinfacht NOx-Sensoren oder Stickoxid-Sensoren bezeichnet werden, sind beispielsweise in Reif, K., Deitsche, K-H. et al., Kraftfahrtechnisches Taschenbuch, Springer Vieweg, Wiesbaden, 2014, Seite 1338-1347 beschrieben.

Stickoxid-Sensoren (= NOx-Sensoren), die heutzutage in der Automobiltechnik eingesetzt werden, funktionieren nach dem Grenzstromprinzip, analog zu Sauerstoff-Sensoren, wie beispielsweise Lambda Sensoren. Ein solcher Stickoxid-Sensor umfasst eine Nernst-Konzentrationszelle, die auch Referenzzelle genannt wird, eine modifizierte Sauerstoffpumpzelle und eine weitere modifizierte Sauerstoffpumpzelle, die sogenannte NOx-Zelle. Eine dem Abgas ausgesetzte äußere Pumpelektrode und eine innere Pumpelektrode in einem ersten Hohlraum, der vom Abgas durch eine Diffusionsbarriere getrennt ist, bilden die Sauerstoffpumpzelle. Im ersten Hohlraum befindet sich auch die Nernstelektrode und in einem Referenzgasraum die Referenzelektrode, die zusammen die Nernstzelle bzw. Referenzzelle bilden. Die NOx-Zelle umfasst eine NOx-Pumpelektrode und eine Gegenelektrode. Die NOx-Pumpelektrode befindet sich in einem zweiten Hohlraum, der mit dem ersten inneren Hohlraum verbunden und von diesem durch eine Diffusionsbarriere getrennt ist. Die Gegenelektrode befindet sich in dem Referenzgasraum. Alle Elektroden in dem ersten und zweiten Hohlraum haben einen gemeinsamen Rückleiter.

Bei Betrieb des Stickoxid-Sensor wird der sogenannten O2-Zelle der Sauerstoff aus dem ersten Hohlraum, der über eine Diffusionsbarriere mit dem Abgas verbunden ist, entfernt. Der dadurch resultierende Pumpstrom ist dann proportional zum Sauerstoffgehalt der Umgebungsluft im Messgas- bzw. Abgasstrom. In der NOx-Zelle werden die Stickoxide abgepumpt. Das Stickoxid NOx, in der in den zweiten Hohlraum befindlichen Atmosphäre, wird durch Anlegen einer konstanten Pumpspannung reduziert bzw. abgebaut. Der durch Reduktion oder Abbau der Messgaskomponente in dem zweiten Hohlraum erzeugte Sauerstoff, der vorzugsweise aus der Reduktion des Stickoxids NOx stammt, wird in einen Referenzgasraum abgepumpt. So hat die angelegte Pumpspannung gegen den Widerstand der NOx-Zelle und der Konzentration des Stickoxids NOx bzw. Sauerstoffs einen Pumpstrom zur Folge, der proportional zum Gehalt an Stickoxid NOx bzw. Sauerstoff ist und das NOx-Messsignal darstellt.

Der dabei resultierende Pumpstrom IP2 ist somit ein Maß für die NOx-Konzentration der Umgebungsluft im Messgas- bzw. Abgasstrom. Bei dieser Anordnung ist es wichtig, dass an der Sauerstoffzelle nicht auch die Stickoxide abgepumpt werden, da sonst an der NOx-Zelle kein Signal mehr gemessen werden könnte. Dies wird durch eine Gold-Dotierung der O2-Zelle erreicht. Zusätzlich darf die O2-Zelle nur bei niedrigen Pumpspannungen betrieben werden, da sonst wieder NOx-Moleküle dissoziiert würden.

Trotz der Vorteile der aus dem Stand der Technik bekannten Sensoren und Verfahren zum Betreiben derselben, beinhalten diese noch Verbesserungspotenzial. Die Temperatur des Sensorelements wird durch eine Pulsweitenmodulation (PWM) der Heizerversorgung (Spannung, Strom) gesteuert. Die Spannung des PWMs wird über einen Feldeffekttransistor (FET) direkt von der Versorgungspannung (12V) der Sensorsteuereinheit (SCU) abgegriffen. Dadurch liegt in der An-Phase des PWMs die SCU-Versorgungsspannung des Systems an den Heizmäander des Sensorelements in der Sensor-Probe an. Der Strom an NOx-Messsignal ist sehr klein wie beispielsweise. 4,5 µA bei 1500 ppm NOx und damit auch äußerst empfindlich gegenüber Störungen und Einkopplungen. Aufgrund der baulichen Nähe der Heizmäander zu der NOx Zelle wird während der An-Phase des PWM-Signals durch kapazitive Kopplung und Leckströme ein Strom auf die IP2 Leitung/Zelle eingeprägt. Durch diese Störung wird ein Offset zu dem tatsächlichen NOx Wert messbar. Die Anforderungen der Umweltbehörden verlangen eine kontinuierliche und verlässliche Diagnose von Leitungsunterbrechungen der IP2 Leitung.

Derartige Sensoren und Verfahren sind beispielsweise aus den Offenlegungsschriften DE 10 2015 210 473 A1 und DE 10 2016 209 924 A1 bekannt.

### Offenbarung der Erfindung

Es wird daher ein Verfahren zum Betreiben eines Sensors zum Nachweis mindestens eines Anteils einer Messgaskomponente mit gebundenem Sauerstoff in einem Messgas vorgeschlagen, welches die Nachteile bekannter Verfahren zum Betreiben dieser Sensoren zumindest weitgehend vermeidet und das in Intervallen von mindestens 500 ms eine sichere und kontinuierliche Diagnose erlaubt, ohne die NOx-Messwerte zu beeinflussen bzw. zu stören.

Bei einem erfindungsgemäßen Verfahren zum Betreiben eines Sensors zum Nachweis mindestens eines Anteils einer Messgaskomponente mit gebundenem Sauerstoff in einem Messgas, insbesondere in einem Abgas einer Verbrennungskraftmaschine, wobei der Sensor ein Sensorelement umfasst, wobei das Sensorelement eine erste Pumpzelle, die eine äußere Pumpelektrode und eine innere Pumpelektrode aufweist und die an einem ersten Hohlraum anliegt, welcher mit dem Messgas in Verbindung steht, eine Referenzzelle, welche eine Nernst-Elektrode und eine Referenzelektrode aufweist und die an einem Referenzgasraum anliegt, und eine zweite Pumpzelle, die eine Pumpelektrode und eine Gegenelektrode aufweist und die an einem zweiten Hohlraum anliegt, wird ein elektronisches Steuergerät, das zumindest über einen ersten gesonderten Anschluss für die erste Pumpzelle, einen zweiten gesonderten Anschluss für die zweite Pumpzelle, einen Anschluss für eine Versorgungsspannung und über einen gemeinsamen Anschluss verfügt, mit dem Sensorelement verbunden, wobei die äußere Pumpelektrode mittels einer ersten mittels einer ersten elektrisch leitenden Verbindung mit dem ersten gesonderten Anschluss verbunden wird, wobei die innere Pumpelektrode mittels einer zweiten elektrisch leitenden Verbindung mit dem gemeinsamen Anschluss verbunden wird, wobei die Referenzelektrode mittels einer dritten elektrisch leitenden Verbindung mit dem Anschluss für eine Versorgungsspannung verbunden wird, wobei die Pumpelektrode mittels der zweiten elektrisch leitenden Verbindung mit dem gemeinsamen Anschluss verbunden wird, wobei die Gegenelektrode mittels einer vierten elektrisch leitenden Verbindung mit dem zweiten gesonderten Anschluss verbunden wird, wobei in der vierten elektrisch leitenden Verbindung ein Messwiderstand vorgesehen wird, wobei mittels des Steuergeräts eine Kapazität der zweiten Pumpzelle zum Erzeugen eines Messsignals an dem Messwiderstand für eine vorbestimmte Zeit verändert wird.

Durch eine Änderung der Kapazität der zweiten Pumpzelle wird an der zweiten Pumpzelle eine Stromänderung bewirkt, was wiederum an dem Messwiderstand ein auswertbares Messsignal erzeugt, das die Unterscheidung zwischen einem offenen oder geschlossenen Stromkreis erlaubt. Mit anderen Worten wird ein IP2-Signal durch eine Änderung der Kapazität der zweiten Pumpzelle erzeugt. Damit lässt sich ein offener Stromkreis an der IP2 Leitung der NOx-Zelle auch während des Messbetriebs sicher erkennen. Auch in Betriebszuständen, in denen der Strom (nahezu) gleich null ist, wie beispielsweise bei 0 ppm NOx, kann eine offene IP2-Leitung detektiert werden.

In einer ersten Alternative der Erfindung wird in die Referenzelektrode mittels der dritten elektrisch leitenden Verbindung von dem Anschluss für eine Versorgungsspannung ein vorbestimmter elektrischer Strom eingeprägt, wobei der vorbestimmte elektrische Strom für die vorbestimmte Zeit verändert wird. Beispielsweise treibt eine Stromquelle einen Pumpstrom ICP über die Vs-Leitung in die Referenzelektrode der Nernstzelle. Eine solche Stromquelle hat einen konstanten Ausgangsstrom unabhängig von der Spannung und anliegenden der Last. Die Größe des Sauerstoffpartialdrucks in der gepumpten Referenzkammer ist in etwa proportional zum anliegenden Pumpstrom. Dieser Pumpstrom stellt Elektronen zur Verfügung, um aus den positiv geladenen Sauerstoffionen O²⁺, die von der Nernstelektrode zu der Referenzelektrode überführten Sauerstoffionen O²⁻ wieder in zwei Sauerstoffatome O₂ zusammenzusetzten. Damit wird die Referenzkammer stetig bei λ>1 mit Sauerstoff gefüllt bzw. aufgepumpt. Dies dient dazu, um einen Partialdruckdifferenz zwischen Referenzraum und der Sauerstoffkammer einzustellen. Der Partialdruck ergibt sich aus dem gesetzten Pumpstrom und dem Verlust von Sauerstoff durch Diffusion. Zielwert für die Partialdruckdifferenz ist eine Nernstspannung von üblicherweise 425mV. Durch eine kleine Öffnung kann der überschüssige Druck entweichen. Durch Variation des Pumpstroms wird der Partialdruck der Referenzzelle verändert und dadurch die Kapazität der zweiten Pumpzelle beeinflusst. Dies hat zur Folge, dass ein IP2-Strom getrieben wird, auch wenn keine Stickoxide, Sauerstoff oder Feuchtigkeit in der zweiten Pumpzelle vorhanden sind. Entsprechend wird durch diesen IP2-Strom ein Messsignal am Messwiderstand erzeugt, das zum Unterscheiden zwischen defekter und intakter IP2-Leitung verwendet werden kann.

Bei einer Weiterbildung wird der vorbestimmte elektrische Strom auf einen Wert von 0 µA für die vorbestimmte Zeit verändert. Die Veränderung wird somit durch An- und Abschalten des Pumpstroms zur Referenzelektrode realisiert, was eine deutliche Änderung des IP2-Stroms bewirkt und somit ein deutlich auswertbares Messsignal am Messwiderstand.

In der ersten Alternative der Erfindung wird die vierte elektrisch leitende Verbindung als intakt identifiziert, falls das Messsignal für die vorbestimmte Zeit eine Veränderung aufweist, und wird als defekt identifiziert wird, falls das Messsignal für die vorbestimmte Zeit keine Veränderung aufweist. Ist mit anderen Worten die IP2-Leitung in Ordnung, so wird bei Änderung des Pumpstroms zur Referenzelektrode ein Strom auf der IP2-Leitung messbar sein. Ist die IP2-Leitung getrennt, so wird trotz Änderung des Pumpstroms zur Referenzelektrode kein Strom auf dieser messbar sein. Somit lässt gut zwischen defekter und intakter IP2-Leitug unterscheiden.

In einer zweiten Alternative der Erfindung weist das Sensorelement ein Heizelement auf, wobei das Sensorelement mittels des Heizelements auf eine vorbestimmte Temperatur beheizt wird, wobei die vorbestimmte Temperatur für die vorbestimmte Zeit verändert wird. Durch Variation der Temperatur in dem Sensorelement kann die elektrische Kapazität der zweiten Pumpzelle beeinflusst werden. Durch Erhöhung der Kapazität werden zusätzliche Ladungsträger gebunden, bei Verringerung werden Ladungsträger wieder abgegeben. Dadurch fließt ein Strom auf der IP2-Leitung, der als Messsignal an dem Messwiderstand auswertbar ist.

In der zweiten Alternative der Erfindung wird die vierte elektrisch leitende Verbindung als intakt identifiziert, falls das Messsignal für die vorbestimmte Zeit eine Veränderung aufweist, und wird als defekt identifiziert wird, falls das Messsignal für die vorbestimmte Zeit keine Veränderung aufweist. Wenn somit die IP2-Leitung in Ordnung ist, so wird ein Strom auf dieser messbar sein, und wenn die IP2-Leitung getrennt ist, so wird trotz Änderung der Temperatur kein Strom auf dieser messbar sein. Somit lässt gut zwischen defekter und intakter IP2-Leitug unterscheiden.

Bei einer Weiterbildung wird die vorbestimmte Temperatur für die vorbestimmte Zeit abgesenkt, wobei die vierte elektrisch leitende Verbindung als intakt identifiziert wird, falls das Messsignal für die vorbestimmte Zeit ein Absinken aufweist, und wird als defekt identifiziert wird, falls das Messsignal für die vorbestimmte Zeit keine Veränderung aufweist.

Alternativ wird die vorbestimmte Temperatur für die vorbestimmte Zeit erhöht, wobei die vierte elektrisch leitende Verbindung als intakt identifiziert wird, falls das Messsignal für die vorbestimmte Zeit einen Anstieg aufweist, und wird als defekt identifiziert wird, falls das Messsignal für die vorbestimmte Zeit keine Veränderung aufweist. Bei kurzzeitiger Verringerung der Temperatur, was eine Erhöhung des Innenwiderstandes des Sensorelements bewirkt, hat dies eine negative Stromänderung zur Folge. Wird die Temperatur dagegen erhöht, was eine Verringerung des Innenwiderstandes des Sensorelements bewirkt, hat dies eine positive Stromänderung zur Folge. Daraus folgt, dass in der zweiten Pumpzelle ein Strom getrieben wird, auch wenn keine Stickoxide, Sauerstoff oder Feuchtigkeit in der zweiten Pumpzelle vorhanden sind. Entsprechend wird durch diesen IP2-Strom ein Messsignal am Messwiderstand erzeugt, das zum Unterscheiden zwischen defekter und intakter IP2-Leitung verwendet werden kann.

Bei einer Weiterbildung wird die Temperatur des Sensorelements durch Erfassen eines elektrischen Innenwiderstands des Sensorelements bestimmt. Durch Messung des Innenwiderstands des Sensorelements kann auf die Temperatur geschlossen werden, da dieser temperaturabhängig ist.

Bei einer Weiterbildung ist das Messsignal ein Spannungsabfall über den Messwiderstand.

Dadurch kann ein gut von dem Steuergerät verarbeitbares Signal gewonnen werden.

Unter einem Festelektrolyten ist im Rahmen der vorliegenden Erfindung ein Körper oder Gegenstand mit elektrolytischen Eigenschaften, also mit Ionen leitenden Eigenschaften, zu verstehen. Insbesondere kann es sich um einen keramischen Festelektrolyten handeln. Dies umfasst auch das Rohmaterial eines Festelektrolyten und daher die Ausbildung als so genannter Grünling oder Braunling, der erst nach einem Sintern zu einem Festelektrolyten wird. Insbesondere kann der Festelektrolyt als Festelektrolytschicht oder aus mehreren Festelektrolytschichten ausgebildet sei. Unter einer Schicht ist im Rahmen der vorliegenden Erfindung eine einheitliche Masse in flächenhafter Ausdehnung einer gewissen Höhe zu verstehen, die über, unter oder zwischen anderen Elementen liegt.

Unter einer Elektrode ist im Rahmen der vorliegenden Erfindung allgemein ein Element zu verstehen, welches in der Lage ist, den Festelektrolyten derart zu kontaktieren, dass durch den Festelektrolyten und die Elektrode ein Strom aufrechterhalten werden kann. Dementsprechend kann die Elektrode ein Element umfassen, an welchem die Ionen in den Festelektrolyten eingebaut und/oder aus dem Festelektrolyten ausgebaut werden können. Typischerweise umfassen die Elektroden eine Edelmetallelektrode, welche beispielsweise als Metall-Keramik-Elektrode auf dem Festelektrolyten aufgebracht sein kann oder auf andere Weise mit dem Festelektrolyten in Verbindung stehen kann. Typische Elektrodenmaterialien sind Platin-Cermet-Elektroden. Auch andere Edelmetalle, wie beispielsweise Gold oder Palladium, sind jedoch grundsätzlich einsetzbar.

Unter einem Heizelement ist im Rahmen der vorliegenden Erfindung ein Element zu verstehen, das zum Erwärmen des Festelektrolyten und der Elektroden auf mindestens ihre Funktionstemperatur und vorzugsweise auf ihre Betriebstemperatur dient. Die Funktionstemperatur ist diejenige Temperatur, ab der der Festelektrolyt für Ionen leitend wird und die ungefähr 350 °C beträgt. Davon ist die Betriebstemperatur zu unterscheiden, die diejenige Temperatur ist, bei der das Sensorelement üblicherweise betrieben wird und die höher ist als die Funktionstemperatur. Die Betriebstemperatur kann beispielsweise von 700 °C bis 950 °C sein. Das Heizelement kann einen Heizbereich und mindestens eine Zuleitungsbahn umfassen. Unter einem Heizbereich ist im Rahmen der vorliegenden Erfindung der Bereich des Heizelements zu verstehen, der in dem Schichtaufbau entlang einer zu der Oberfläche des Sensorelements senkrechten Richtung mit einer Elektrode überlappt. Üblicherweise erwärmt sich der Heizbereich während des Betriebs stärker als die Zuleitungsbahn, so dass diese unterscheidbar sind. Die unterschiedliche Erwärmung kann beispielsweise dadurch realisiert werden, dass der Heizbereich einen höheren elektrischen Widerstand aufweist als die Zuleitungsbahn. Der Heizbereich und/oder die Zuleitung sind beispielsweise als elektrische Widerstandsbahn ausgebildet und erwärmen sich durch Anlegen einer elektrischen Spannung. Das Heizelement kann beispielsweise aus einem Platin-Cermet hergestellt sein. Die Erfindung ist direkt durch eine verkürzte Wartezeit bis zum Erreichen der Betriebsbereitschaft nach Start des Sensors nachweisbar. Die jeweiligen Potenziale können an den Zuleitungen gemessen werden.

Unter einer Kapazität der zweiten Pumpzelle ist im Rahmen der vorliegenden Erfindung eine physikalische Größe zu verstehen, die zwischen zwei voneinander isolierten elektrisch leitenden Körpern besteht und gleich dem Verhältnis der Ladungsmenge, die auf diesen Leitern gespeichert ist, und der zwischen ihnen herrschenden elektrischen Spannung ist. Die elektrisch leitenden Körper sind Elektroden und die Isolierung zwischen diesen wird durch den Festelektrolyten bewirkt. Entsprechend kann man die zweite Pumpzelle als einen Kondensator ansehen.

Die Erfindung hinsichtlich der Diagnose der IP2-Leitung durch Änderung des Pumpstroms zur Referenzelektrode ist gut und einfach nachweisbar, da Änderungen des Pumpstroms zur Referenzelektrode und das gewünschte Diagnosesignal auf der IP2-Leitung einfach messbar sind. Mit einem Oszilloskop und empfindlichen Tastköpfen kann der Pumpstroms zur Referenzelektrode auf der Vs-Leitung und der IP2-Strom auf der IP2-Leitung einfach gemessen werden. Zudem kann die Kopplung des Pumpstroms zur Referenzelektrode auf das O2-Signal gemessen werden.

Die Erfindung hinsichtlich der Diagnose der IP2-Leitung durch Änderung der Temperatur des Sensorelements ist ebenfalls gut und einfach nachweisbar, da eine systematische oder sich wiederholende Temperaturänderung zum Beispiel mit einem Oszilloskop an der Heizerleitung während des Messbetriebs, durch Beobachtung der Pulsweitenmodulation, messbar ist. Dabei ist die Temperaturänderung nachweisbar, wenn das Tastverhältnis der Pulsweitenmodulation um eine signifikante Länge von dem vorangehenden oder nachfolgenden Tastverhältnis abweicht Dabei kann dieses entweder größer oder kleiner sein. Dies ist besonders bei stabilen Umgebungstemperaturverhältnissen an dem Sensor gut nachweisbar. D.h. es dürfen keine Temperaturänderungen und keine Luftbewegungen an dem Sensor auftreten. Des Weiteren ist die Temperaturänderung auch durch Temperaturmessungen mit z.B. einem Infrarot-Messgerät an der Keramik des Sensors nachweisbar. Wird an dem Sensor keine Pulsweitenmodulation, sondern eine Gleichspannung zur Regelung der Temperaturverwendet, dann ist ein Hinweis auf die Verwendung, wenn die Spannung kurzzeitig erhöht oder abgesenkt wird.

### Kurze Beschreibung der Zeichnungen

Weitere optionale Einzelheiten und Merkmale der Erfindung ergeben sich aus der nachfolgenden Beschreibung bevorzugter Ausführungsbeispiele, welche in den Figuren schematisch dargestellt sind.

Es zeigen:
Figur 1 einen prinzipiellen Aufbau eines erfindungsgemäßen Sensors,
Figur 2 einen Teil des Sensors mit einem Teil eines daran angeschlossenen Steuergeräts,
Figur 3 einen weiteren Teil des Sensors mit einem Teil eines daran angeschlossenen Steuergeräts,
Figur 4 ein erstes Beispiel für einen zeitlichen Verlauf von elektrischen Spannungen und Messsignal bei dem Sensor und
Figur 5 ein zweites Beispiel für einen zeitlichen Verlauf von elektrischen Spannungen und Messsignal bei dem Sensor.

### Ausführungsformen der Erfindung

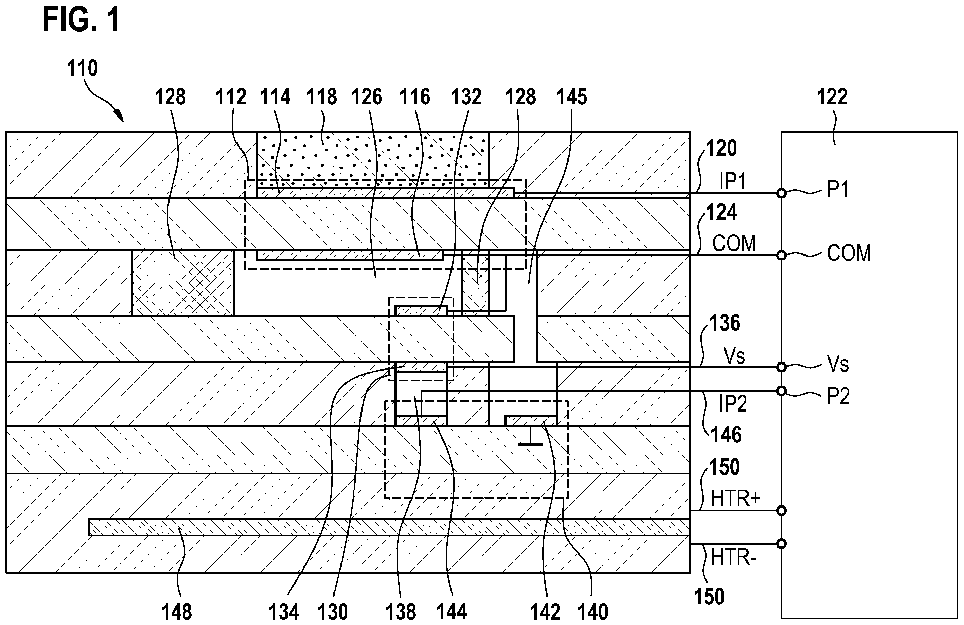

Figur 1 zeigt einen prinzipiellen Aufbau eines erfindungsgemäßen Sensors 100, welcher zur Durchführung des erfindungsgemäßen Verfahrens besonders geeignet ist.

Der Sensor 100, welcher zum Nachweis mindestens eines Anteils einer Messgaskomponente mit gebundenem Sauerstoff, im Folgenden beispielhaft als Stickoxid NOx bezeichnet, in einem Gasgemisch, beispielhaft einem Abgas einer Verbrennungskraftmaschine, eingerichtet ist, umfasst hierzu ein Sensorelement 110 eine erste Pumpzelle 112, welche zwischen einer äußeren Pumpelektrode 114 und einer inneren Pumpelektrode 116 ausgebildet ist. Die äußere Pumpelektrode 114, welche mittels einer porösen Aluminiumoxidschicht 118 von der Umgebung des Sensors 100 getrennt ist, verfügt hierbei über eine erste elektrisch leitende Verbindung 120, über welche sich ein erster Pumpstrom I_{P1} in der ersten Pumpzelle 112 erzeugen lässt. Die erste elektrisch leitende Verbindung 120 ist hierzu mit einem ersten Anschluss P1 eines externen elektronischen Steuergeräts 122 verbunden. Um einen vollständigen Stromkreis zu erhalten, verfügt die innere Pumpelektrode 116 ebenfalls über eine zweite elektrisch leitende Verbindung 124, welche zu einem gemeinsamen Anschluss COM des externen elektronischen Steuergeräts 122 führt. Die erste Pumpzelle 112 liegt an einem ersten Hohlraum 126 an, der sich im Inneren des Sensorelements 110 befindet und mit dem Messgas in Verbindung steht. Durch Erzeugen des ersten Pumpstroms I_{P1} in der ersten Pumpzelle 112 lässt sich ein erster Anteil von Sauerstoffionen, welche aus molekularem Sauerstoff aus dem Gasgemisch gebildet werden, zwischen dem ersten Hohlraum 126 und der Umgebung des Sensors 100 transportieren. In dem Eintrittsweg aus der Umgebung zu dem ersten Hohlraum 126 sind zwei Diffusionsbarrieren 128 vorhanden.

Das Sensorelement 110 weist weiterhin eine elektrische Referenzzelle 130 auf, welche eine Nernst-Elektrode 132 und eine Referenzelektrode 134 aufweist. Während die Nernst-Elektrode 132 über die zweite elektrisch leitende Verbindung 124 zusammen mit der inneren Pumpelektrode 116 zu dem gemeinsamen Anschluss COM verfügt, weist die Referenzelektrode 134 eine gesonderte dritte elektrisch leitende Verbindung 136 zu einer Versorgungsspannung Vs auf, welche über einen Anschluss Vs des externen elektronischen Steuergeräts 122 die erforderliche Versorgungsspannung Vs bereitstellt. Die Referenzzelle 130 liegt an einem Referenzgasraum 138 an. Ein zweiter Anteil der Sauerstoffionen aus dem ersten Hohlraum 126 und/oder aus der Umgebung des Sensors 100 wird in den Referenzgasraum 138 durch Anlegen eines Referenz-Pumpstroms zwischen dem Anschluss Vs und dem gemeinsamen Anschluss COM transportiert. Hierbei wird der Wert für den Referenz-Pumpstrom derart eingestellt, dass sich ein festgelegter Anteil der Sauerstoffionen in dem Referenzgasraum 138 ausbildet. Vorzugsweise wird in diesem Zusammenhang auch der Wert für den ersten Pumpstrom I_{P1} derart eingestellt, dass sich ein festgelegtes Verhältnis zwischen dem ersten Anteil der Sauerstoffionen in dem ersten Hohlraum 126 und dem zweiten Anteil der Sauerstoffionen in dem Referenzgasraum 138 ergibt.

Die in dem Gasgemisch weiterhin enthaltene Messgaskomponente Stickoxid NOₓ mit dem gebundenen Sauerstoff gelangt, insbesondere durch Diffusion, weitgehend unbeeinflusst in eine zweite Pumpzelle 140 des Sensorelements 110, welche auch als "NOₓ-Pumpzelle" bezeichnet werden kann. Die zweite Pumpzelle 140 weist eine NOₓ-Pumpelektrode 142 und eine NOₓ-Gegenelektrode 144 auf und liegt an einem zweiten Hohlraum 145 im Inneren des Sensorelements 110 an. Der zweite Hohlraum 145 ist von dem ersten Hohlraum 126 durch eine der Diffusionsbarrieren 128 getrennt. Wenigstens eine der beiden Elektroden NOₓ-Pumpelektrode 142 und/oder NOₓ-Gegenelektrode 144 sind derart ausgestaltet, dass bei Anlegen einer Spannung mittels Katalyse aus der Messgaskomponente NOₓ weiterer molekularer Sauerstoff erzeugt werden kann, welcher in der zweiten Pumpzelle 140 gebildet wird.

Während die NOₓ-Pumpelektrode 142 eine elektrisch leitende Verbindung aufweist, welche zu dem gemeinsamen Anschluss COM führt, weist die NOₓ-Gegenelektrode 144 eine vierte elektrisch leitende Verbindung 146 auf, über welche ein zweiter Pumpstrom I_{P2} an die zweite Pumpzelle 140 angelegt werden kann. Die vierte elektrisch leitende Verbindung 146 ist hierzu mit einem zweiten Anschluss P2 des externen elektronischen Steuergeräts 122 verbunden. Bei Anlegen eines zweiten Pumpstroms I_{P2} an die zweite Pumpzelle 140 wird ein Anteil von weiteren Sauerstoffionen, welche aus dem weiteren molekularen Sauerstoff gebildet wurden, in den Referenzgasraum 138 transportiert.

Das Sensorelement 110 verfügt weiterhin über ein Heizelement 148, welches eine Heizleitung 150 mit den Leitungen HTR+ und HTR- aufweist, über welche ein Heizstrom in das Heizelement 148 eingebracht werden kann, welches mittels Erzeugen einer Heizleistung das Sensorelement 110 auf die gewünschte Temperatur bringen kann.

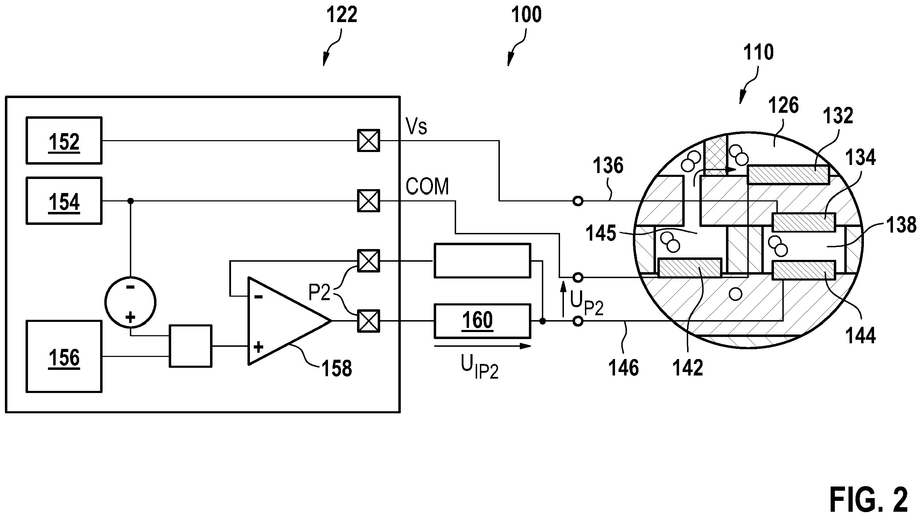

Figur 2 zeigt einen Teil des Sensors 100 mit einem Teil eines daran angeschlossenen Steuergeräts 122. Das Steuergerät 122 weist einen Analog-Digital-Wandler 152 auf, der mit dem Anschluss Vs für die Versorgungsspannung verbunden ist. Das Steuergerät 122 weist weiterhin eine COM-Spannungsquelle 154 auf, die mit dem gemeinsamen Anschluss COM verbunden ist. Das Steuergerät 122 weist weiterhin eine Anregungssignalquelle 156 auf, die mit dem Pluspol eines Operationsverstärkers 158 verbunden ist. Bei dem gezeigten Ausführungsbeispiel ist der Operationsverstärker 158 ein Spannungsfolger. Die COM-Spannungsquelle 154 ist ebenfalls mit dem Pluspol des Operationsverstärkers 158 verbunden. Der Operationsverstärker 158 ist wiederum mit dem zweiten Anschluss P2 verbunden. In der vierten elektrischen Leitung 146 ist zwischen dem zweiten Anschluss P2 und der Gegenelektrode 144 ein Messwiderstand 160 angeordnet.

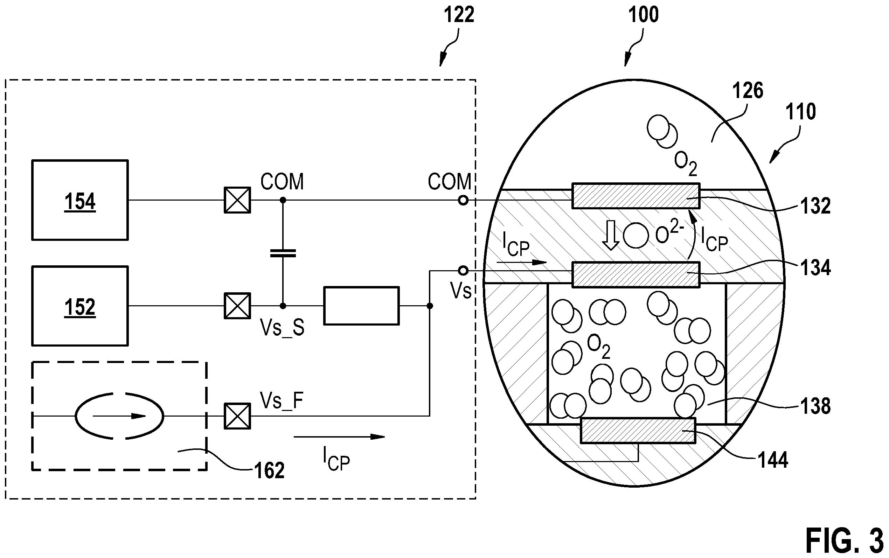

Figur 3 zeigt einen weiteren Teil des Sensors 100 mit einem Teil eines daran angeschlossenen Steuergeräts 122. Gezeigt ist die COM-Spannungsquelle 154, die mit dem gemeinsamen Anschluss COM verbunden ist, der wiederum über die zweite elektrisch leitende Verbindung 124 mit der Nernstelektrode 132 verbunden. Gezeigt ist weiterhin der Analog-Digital-Wandler 152, der mit dem Anschluss Vs für die Versorgungsspannung verbunden ist, der wiederum über die dritte elektrisch leitende Verbindung 136 mit der Referenzelektrode 134 verbunden ist. Das Steuergerät 122 weist weiterhin eine Stromquelle 162 auf. Die Stromquelle 162 ist ebenfalls mit dem Anschluss Vs für die Versorgungsspannung verbunden. Die Stromquelle 162 ist ausgebildet, mittels der dritten elektrisch leitenden Verbindung 136 von dem Anschluss Vs für die Versorgungsspannung in die Referenzelektrode 134 einen vorbestimmten elektrischen Strom I_{CP} einzuprägen. Mit anderen Worten wird mittels der Stromquelle 162 ein Pumpstrom I_{CP} in die Referenzelektrode 134 getrieben. Die Stromquelle 162 hat einen konstanten Ausgangsstrom unabhängig von der Spannung und der anliegenden Last. Der vorbestimmte elektrische Strom I_{CP} ist ein Wert in einem Bereich von 3 µA bis 6 µA. Die Größe des Sauerstoffpartialdrucks in dem gepumpten Referenzgasraum 138 ist in etwa proportional zum anliegenden Pumpstrom I_{CP}. Dieser Pumpstrom I_{CP} stellt Elektronen zur Verfügung, um aus den positiv geladenen Sauerstoffionen O²⁺, die von der Nernstelektrode 132 zu der Referenzelektrode 134 überführten Sauerstoffionen O²⁻ wieder in zwei Sauerstoffatome O₂ zusammenzusetzten. Damit wird der Referenzgasraum 138 stetig bei λ>1 mit Sauerstoff gefüllt bzw. aufgepumpt. Dies dient dazu, um einen Partialdruckdifferenz zwischen Referenzgasraum 138 und ersten Hohlraum 126 einzustellen. Der Partialdruck ergibt sich aus dem gesetzten Pumpstrom I_{CP} und dem Verlust von Sauerstoff durch Diffusion. Zielwert für die Partialdruckdifferenz ist eine Nernstspannung von üblicherweise 425mV.

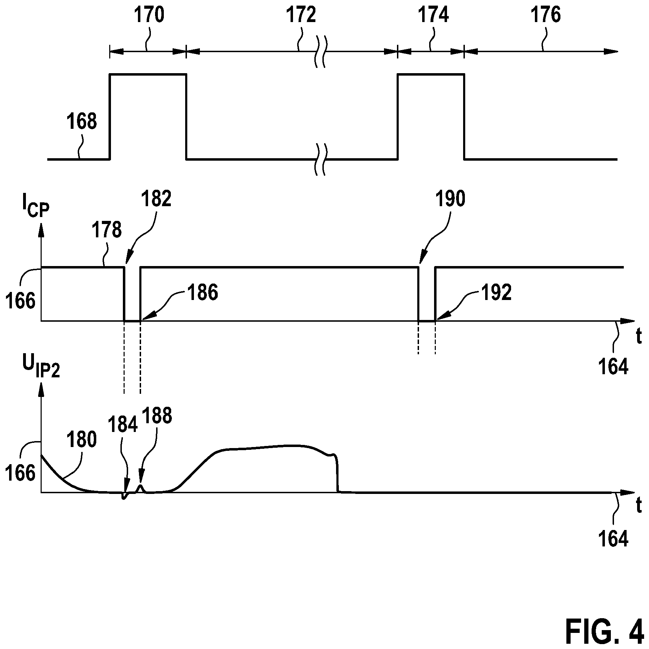

Figur 4 zeigt ein erstes Beispiel für einen zeitlichen Verlauf von elektrischen Spannungen und Messsignal bei dem Sensor 100. Auf der X-Achse 164 ist die Zeit aufgetragen. Auf der Y-Achse 166 sind von oben nach unten gesehen die Heizspannung des Heizelements 148, der in die Referenzelektrode 134 eingeprägte elektrische Strom I_{CP} und der Spannungsabfall U_{IP2} am Messwiderstand 160 aufgetragen. Der Spannungsabfall U_{IP2} am Messwiderstand 160 stellt das Messsignal dar. Die Temperatur des Sensorelements 110 wird durch eine Pulsweitenmodulation (PWM) der Heizerversorgung (Spannung bzw. Strom) gesteuert. Entsprechend zeigt eine Kurve 168, die die Heizspannung des Heizelements 148 darstellt, zumindest eine erste Phase bzw. einen ersten Zeitraum 170, in dem an das Heizelement 148 eine elektrische Spannung angelegt ist und das Heizelement 148 somit angeschaltet ist, und eine zweite Phase bzw. einen zweiten Zeitraum 172, in dem an das Heizelement 148 keine elektrische Spannung angelegt ist und das Heizelement 148 somit ausgeschaltet ist. Dabei folgt der zweite Zeitraum 172 auf den ersten Zeitraum 170. Eine Summe aus erstem Zeitraum 170 und zweitem Zeitraum 172 ist beispielsweise 500 ms. Bei dem gezeigten Ausführungsbeispiel zeigt die Kurve 168 weiterhin eine dritte Phase bzw. einen dritten Zeitraum 174, in dem an das Heizelement 148 eine elektrische Spannung angelegt ist und das Heizelement 148 somit angeschaltet ist, und eine vierte Phase bzw. einen vierten Zeitraum 176, in dem an das Heizelement 148 keine elektrische Spannung angelegt ist und das Heizelement 148 somit ausgeschaltet ist. Dabei folgt der vierte Zeitraum 176 auf den dritten Zeitraum 174. Eine Summe aus drittem Zeitraum 174 und viertem Zeitraum 176 ist beispielsweise 500 ms.

Zur Durchführung einer Diagnose der vierten elektrischen Leitung 146, die den zweiten gesonderten Anschluss P2 mit der Pumpzelle 140 bzw. der Gegenelektrode 144 verbindet, wird mittels des Steuergeräts 122 eine Kapazität der zweiten Pumpzelle 140 zum Erzeugen eines Messsignals an dem Messwiderstand 160 für eine vorbestimmte Zeit verändert. So wird in die Referenzelektrode 134 mittels der dritten elektrisch leitenden Verbindung 136 von dem Anschluss für eine Versorgungsspannung Vs ein vorbestimmter elektrische Strom I_{CP}, dargestellt durch die Kurve 178, eingeprägt. Dieser vorbestimmte elektrische Strom I_{CP} wird für die vorbestimmte Zeit verändert. Bei dem gezeigten Ausführungsbeispiel wird der vorbestimmte elektrische Strom I_{CP} auf einen Wert von 0 µA für die vorbestimmte Zeit verändert. Dies geschieht in einem Zeitraum, in dem das Heizelement 148 angeschaltet ist, beispielsweise in dem ersten Zeitraum 170 und dem dritten Zeitraum 174. Durch Veränderung des vorbestimmten elektrischen Stroms I_{CP} wird der Partialdruck der Referenzzelle 130 verändert und dadurch die Kapazität der zweiten Pumpzelle 140 beeinflusst. Dies hat zur Folge, dass ein Strom I_{P2} durch die vierte elektrisch leitende Verbindung 146 getrieben wird. Dies erzeugt ein Messsignal 180 an dem Messwiderstand 160. Die vierte elektrisch leitende Verbindung 146 wird als intakt identifiziert, falls das Messsignal 180 für die vorbestimmte Zeit eine Veränderung aufweist, und wird als defekt identifiziert wird, falls das Messsignal 180 für die vorbestimmte Zeit keine Veränderung aufweist. Bei dem gezeigten Ausführungsbeispiel weist das Messsignal 180 beim Abschalten des vorbestimmten elektrischen Stroms I_{CP}, d.h. beim Ändern auf 0 µA, zum Zeitpunkt 182 in dem ersten Zeitraum 170 einen durch einen Pfeil angedeuteten negativen Peak 184 und beim Wiedereinschalten, d.h. beim erneuten Ändern auf den Ausgangswert, zum Zeitpunkt 186 einen durch einen Pfeil angedeuteten positiven Peak 188 auf. In dem ersten Zeitraum 170 weist das Messsignal 180 somit für die vorbestimmte Zeit eine Veränderung auf. Dies bedeutet, dass in dem ersten Zeitraum 170 die vierte elektrisch leitende Verbindung 146 als intakt identifiziert wird, da durch die vierte elektrisch leitende Verbindung 146 ein Strom I_{P2} getrieben wird. Dahingegen weist das Messsignal 180 in dem dritten Zeitraum 174 beim Abschalten des vorbestimmten elektrischen Stroms I_{CP}, d.h. beim Ändern auf 0 µA, zum Zeitpunkt 190 und beim Wiedereinschalten, d.h. beim erneuten Ändern auf den Ausgangswert, zum Zeitpunkt 192 keine Veränderung auf. Dies bedeutet, dass in dem dritten Zeitraum 174 bei Veränderung vorbestimmten elektrischen Stroms I_{CP} kein Strom I_{P2} durch die vierte elektrisch leitende Verbindung 146 getrieben wird, was auf eine Unterbrechung der vierten elektrischen Leitung 146 hindeutet.

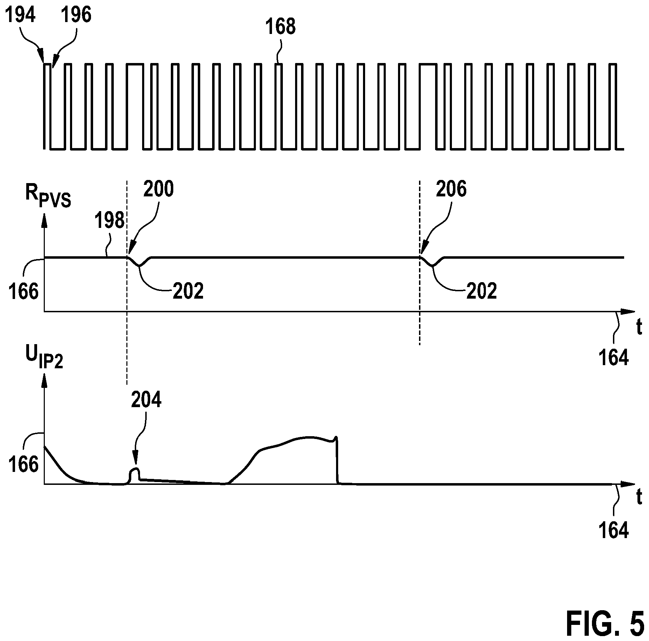

Figur 5 zeigt ein zweites Beispiel für einen zeitlichen Verlauf von elektrischen Spannungen und Messsignal bei dem Sensor 100. Auf der X-Achse 164 ist die Zeit aufgetragen. Auf der Y-Achse 166 sind von oben nach unten gesehen die Heizspannung des Heizelements 148, der elektrische Innenwiderstand des Sensorelements 110 und der Spannungsabfall U_{IP2} am Messwiderstand 160 aufgetragen. Der Spannungsabfall U_{IP2} am Messwiderstand 160 stellt das Messsignal dar. Die Temperatur des Sensorelements 110 wird durch eine Pulsweitenmodulation (PWM) der Heizerversorgung (Spannung bzw. Strom) gesteuert. Entsprechend zeigt eine Kurve 168, die die Heizspannung des Heizelements 148 darstellt, Phasen bzw. Zeiträume 194, in denen an das Heizelement 148 eine elektrische Spannung angelegt ist und das Heizelement 148 somit angeschaltet ist, und Phasen bzw. Zeiträume 196, in denen an das Heizelement 148 keine elektrische Spannung angelegt ist und das Heizelement 148 somit ausgeschaltet ist. Die Temperatur des Sensorelements 110 wird durch Erfassen eines elektrischen Innenwiderstands R_{PVS} des Sensorelements 110 bestimmt. Entsprechend stellt die Kurve 198 den elektrischen Innenwiderstand R_{PVS} des Sensorelements 110 dar.

Zur Durchführung einer Diagnose der vierten elektrischen Leitung 146, die den zweiten gesonderten Anschluss P2 mit der Pumpzelle 140 bzw. der Gegenelektrode 144 verbindet, wird mittels des Steuergeräts 122 eine Kapazität der zweiten Pumpzelle 140 zum Erzeugen eines Messsignals an dem Messwiderstand 160 für eine vorbestimmte Zeit verändert. So wird das Sensorelement 110 mittels des Heizelements 148 auf eine vorbestimmte Temperatur beheizt. Die vorbestimmte Temperatur entspricht beispielsweise einem elektrischen Innenwiderstand R_{PVS} des Sensorelements 110 von 300 Ohm. Diese vorbestimmte Temperatur wird für die vorbestimmte Zeit verändert. Durch Variation der Temperatur in dem Sensorelement 110 kann die elektrische Kapazität der zweiten Pumpzelle 140 beeinflusst werden. Durch Erhöhung der Kapazität werden zusätzliche Ladungsträger gebunden, bei Verringerung werden Ladungsträger wieder abgegeben. Dadurch fließt ein Strom I_{P2} auf der vierten elektrisch leitenden Verbindung 146. Bei kurzzeitiger Verringerung der Temperatur, was eine Erhöhung des Innenwiderstandes des Sensorelements 110 bewirkt, hat dies eine negative Stromänderung zur Folge. Wird die Temperatur dagegen erhöht, was eine Verringerung des Innenwiderstandes des Sensorelements 110 bewirkt, hat dies eine positive Stromänderung zur Folge. Daraus folgt, dass in der zweiten Pumpzelle 140 ein Strom I_{P2} getrieben wird, auch wenn keine Stickoxide, Sauerstoff oder Feuchtigkeit in der zweiten Pumpzelle 140 vorhanden sind. Wenn die vierte elektrisch leitende Verbindung 146 intakt ist, so wird ein Strom auf der vierten elektrisch leitenden Verbindung 146 messbar sein, und wenn die vierte elektrisch leitende Verbindung 146 getrennt ist, so wird trotz Änderung der Temperatur kein Strom auf dieser messbar sein. Entsprechend wird die vierte elektrisch leitende Verbindung 146 als intakt identifiziert, falls das Messsignal 180 für die vorbestimmte Zeit eine Veränderung aufweist, und wird als defekt identifiziert wird, falls das Messsignal 180 für die vorbestimmte Zeit keine Veränderung aufweist. Bei dem gezeigten Ausführungsbeispiel wird zum Zeitpunkt 200 die vorbestimmte Temperatur für die vorbestimmte Zeit erhöht, was sich in einem Absinken 202 der Kurve 198, die den elektrischen Innenwiderstand R_{PVS} des Sensorelements 110 darstellt, zeigt. In diesem Fall wird die vierte elektrisch leitende Verbindung 146 als intakt identifiziert, falls das Messsignal 180 für die vorbestimmte Zeit einen Anstieg 204 aufweist. Zum Zeitpunkt 200 weist das Messsignal 180 den Anstieg 204 auf, so dass die vierte elektrisch leitende Verbindung 146 zum Zeitpunkt 202 als intakt identifiziert wird. Bei dem gezeigten Ausführungsbeispiel wird zum Zeitpunkt 206 die vorbestimmte Temperatur ebenfalls für die vorbestimmte Zeit erhöht, was sich in einem Absinken 202 der Kurve 198, die den elektrischen Innenwiderstand R_{PVS} des Sensorelements 110 darstellt, zeigt. In diesem Fall wird die vierte elektrisch leitende Verbindung 146 als defekt identifiziert, falls das Messsignal 180 für die vorbestimmte Zeit keine Veränderung aufweist. Zum Zeitpunkt 206 weist das Messsignal 180 keine Veränderung trotz der Erhöhung der Temperatur auf, so dass die vierte elektrisch leitende Verbindung 146 zum Zeitpunkt 206 als defekt identifiziert wird. Alternativ wird die vorbestimmte Temperatur für die vorbestimmte Zeit abgesenkt. In diesem Fall wird die vierte elektrisch leitende Verbindung 146 als intakt identifiziert, falls das Messsignal 180 für die vorbestimmte Zeit ein Absinken aufweist, und wird als defekt identifiziert, falls das Messsignal 180 für die vorbestimmte Zeit keine Veränderung aufweist.

## Patentansprüche

1. Verfahren zum Betreiben eines Sensors (100) zum Nachweis mindestens eines Anteils einer Messgaskomponente mit gebundenem Sauerstoff in einem Messgas, insbesondere in einem Abgas einer Verbrennungskraftmaschine, umfassend ein Sensorelement (110), wobei das Sensorelement (110) eine erste Pumpzelle (112), die eine äußere Pumpelektrode (114) und eine innere Pumpelektrode (116) aufweist und die an einem ersten Hohlraum (126) anliegt, welcher mit dem Messgas in Verbindung steht, eine Referenzzelle (130), welche eine Nernst-Elektrode (132) und eine Referenzelektrode (134) aufweist und die an einem Referenzgasraum (138) anliegt, und eine zweite Pumpzelle (140), die eine Pumpelektrode (142) und eine Gegenelektrode (144) aufweist und die an einem zweiten Hohlraum (145) anliegt, wobei sich die Gegenelektrode (144) der zweiten Pumpzelle (140) im Referenzgasraum (138) befindet, wobei ein elektronisches Steuergerät (122), das zumindest über einen ersten gesonderten Anschluss (P1) für die erste Pumpzelle (112), einen zweiten gesonderten Anschluss (P2) für die zweite Pumpzelle (140), einen Anschluss für eine Versorgungsspannung (Vs) und über einen gemeinsamen Anschluss (COM) verfügt, mit dem Sensorelement (110) verbunden wird, wobei die äußere Pumpelektrode (114) mittels einer ersten mittels einer ersten elektrisch leitenden Verbindung (120) mit dem ersten gesonderten Anschluss (P1) verbunden wird, wobei die innere Pumpelektrode (116) mittels einer zweiten elektrisch leitenden Verbindung (124) mit dem gemeinsamen Anschluss (COM) verbunden wird, wobei die Referenzelektrode (134) mittels einer dritten elektrisch leitenden Verbindung (136) mit dem Anschluss für eine Versorgungsspannung (Vs) verbunden wird, wobei die Pumpelektrode (142) mittels der zweiten elektrisch leitenden Verbindung (124) mit dem gemeinsamen Anschluss (COM) verbunden wird, wobei die Gegenelektrode (144) mittels einer vierten elektrisch leitenden Verbindung (146) mit dem zweiten gesonderten Anschluss (P2) verbunden wird, wobei in der vierten elektrisch leitenden Verbindung (146) ein Messwiderstand (160) vorgesehen wird, wobei mittels des Steuergeräts (122) eine Kapazität der zweiten Pumpzelle (140) zum Erzeugen eines Messsignals (180) an dem Messwiderstand (160) für eine vorbestimmte Zeit verändert wird, wobei in die Referenzelektrode (134) mittels der dritten elektrisch leitenden Verbindung (136) von dem Anschluss für eine Versorgungsspannung (Vs) ein vorbestimmter elektrischer Strom (I_{CP}) eingeprägt wird, wobei der vorbestimmte elektrische Strom (I_{CP}) für die vorbestimmte Zeit verändert wird, wobei die vierte elektrisch leitende Verbindung (146) als intakt identifiziert wird, falls das Messsignal (180) für die vorbestimmte Zeit eine Veränderung aufweist, und wird als defekt identifiziert, falls das Messsignal (180) für die vorbestimmte Zeit keine Veränderung aufweist.

2. Verfahren nach Anspruch 1, wobei der vorbestimmte elektrische Strom (I_{CP}) auf einen Wert von 0 µA für die vorbestimmte Zeit verändert wird.

3. Verfahren zum Betreiben eines Sensors (100) zum Nachweis mindestens eines Anteils einer Messgaskomponente mit gebundenem Sauerstoff in einem Messgas, insbesondere in einem Abgas einer Verbrennungskraftmaschine, umfassend ein Sensorelement (110), wobei das Sensorelement (110) eine erste Pumpzelle (112), die eine äußere Pumpelektrode (114) und eine innere Pumpelektrode (116) aufweist und die an einem ersten Hohlraum (126) anliegt, welcher mit dem Messgas in Verbindung steht, eine Referenzzelle (130), welche eine Nernst-Elektrode (132) und eine Referenzelektrode (134) aufweist und die an einem Referenzgasraum (138) anliegt, und eine zweite Pumpzelle (140), die eine Pumpelektrode (142) und eine Gegenelektrode (144) aufweist und die an einem zweiten Hohlraum (145) anliegt, wobei sich die Gegenelektrode (144) der zweiten Pumpzelle (140) im Referenzgasraum (138) befindet, wobei ein elektronisches Steuergerät (122), das zumindest über einen ersten gesonderten Anschluss (P1) für die erste Pumpzelle (112), einen zweiten gesonderten Anschluss (P2) für die zweite Pumpzelle (140), einen Anschluss für eine Versorgungsspannung (Vs) und über einen gemeinsamen Anschluss (COM) verfügt, mit dem Sensorelement (110) verbunden wird, wobei die äußere Pumpelektrode (114) mittels einer ersten mittels einer ersten elektrisch leitenden Verbindung (120) mit dem ersten gesonderten Anschluss (P1) verbunden wird, wobei die innere Pumpelektrode (116) mittels einer zweiten elektrisch leitenden Verbindung (124) mit dem gemeinsamen Anschluss (COM) verbunden wird, wobei die Referenzelektrode (134) mittels einer dritten elektrisch leitenden Verbindung (136) mit dem Anschluss für eine Versorgungsspannung (Vs) verbunden wird, wobei die Pumpelektrode (142) mittels der zweiten elektrisch leitenden Verbindung (124) mit dem gemeinsamen Anschluss (COM) verbunden wird, wobei die Gegenelektrode (144) mittels einer vierten elektrisch leitenden Verbindung (146) mit dem zweiten gesonderten Anschluss (P2) verbunden wird, wobei in der vierten elektrisch leitenden Verbindung (146) ein Messwiderstand (160) vorgesehen wird, wobei mittels des Steuergeräts (122) eine Kapazität der zweiten Pumpzelle (140) zum Erzeugen eines Messsignals (180) an dem Messwiderstand (160) für eine vorbestimmte Zeit verändert wird, wobei das Sensorelement (110) ein Heizelement (148) aufweist, wobei das Sensorelement (110) mittels des Heizelements (148) auf eine vorbestimmte Temperatur beheizt wird, wobei die vorbestimmte Temperatur für die vorbestimmte Zeit verändert wird, wobei die vierte elektrisch leitende Verbindung (146) als intakt identifiziert wird, falls das Messsignal (180) für die vorbestimmte Zeit eine Veränderung aufweist, und wird als defekt identifiziert, falls das Messsignal (180) für die vorbestimmte Zeit keine Veränderung aufweist.

4. Verfahren nach Anspruch 3, wobei die vorbestimmte Temperatur für die vorbestimmte Zeit abgesenkt wird, wobei die vierte elektrisch leitende Verbindung (146) als intakt identifiziert wird, falls das Messsignal (180) für die vorbestimmte Zeit ein Absinken aufweist, und wird als defekt identifiziert, falls das Messsignal (168) für die vorbestimmte Zeit keine Veränderung aufweist.

5. Verfahren nach Anspruch 3, wobei die vorbestimmte Temperatur für die vorbestimmte Zeit erhöht wird, wobei die vierte elektrisch leitende Verbindung (146) als intakt identifiziert wird, falls das Messsignal (180) für die vorbestimmte Zeit einen Anstieg aufweist, und wird als defekt identifiziert, falls das Messsignal (180) für die vorbestimmte Zeit keine Veränderung aufweist.

6. Verfahren nach einem der Ansprüche 3 bis 5, wobei die Temperatur des Sensorelements (110) durch Erfassen eines elektrischen Innenwiderstands (R_{PVS}) des Sensorelements (110) bestimmt wird.

7. Verfahren nach einem der Ansprüche 1 bis 6, wobei das Messsignal (180) ein Spannungsabfall (U_{IP2}) über den Messwiderstand (160) ist.

8. Elektronisches Steuergerät, welches über einen ersten gesonderten Anschluss (P1), einen zweiten gesonderten Anschluss (P2), einen Anschluss für eine Versorgungsspannung (Vs) und über einen gemeinsamen Anschluss (COM) verfügt, und über Mittel, die geeignet sind, die Schritte des Verfahrens nach einem der vorangehenden Ansprüchen auszuführen.

9. Computerprogramm, umfassend Befehle, die bewirken, dass ein Steuergerät nach Anspruch 8, die Schritte des Verfahrens nach einem der Ansprüche 1 bis 7 ausführt.

10. Elektronisches Speichermedium, auf welchem ein Computerprogramm nach dem vorhergehenden Anspruch gespeichert ist.

## Claims

1. Method for operating a sensor (100) for detecting at least one portion of a measurement gas component with bound oxygen in a measurement gas, in particular in an exhaust gas of an internal combustion engine, comprising a sensor element (110), wherein the sensor element (110) a first pump cell (112) which has an outer pump electrode (114) and an inner pump electrode (116) and is present at a first cavity (126) connected to the measurement gas, a reference cell (130) which has a Nernst electrode (132) and a reference electrode (134) and is present at a reference gas space (138), and a second pump cell (140) which has a pump electrode (142) and a counter-electrode (144) and is present at a second cavity (145), wherein the counter-electrode (144) of the second pump cell (140) is in the reference gas space (138), wherein an electronic control device (122), which has at least a first separate terminal (P1) for the first pump cell (112), a second separate terminal (P2) for the second pump cell (140), a terminal for a supply voltage (Vs) and a common terminal (COM), is connected to the sensor element (110), wherein the outer pump electrode (114) is connected to the first separate terminal (P1) by means of a first by means of a first electrically conductive connection (120), wherein the inner pump electrode (116) is connected to the common terminal (COM) by means of a second electrically conductive connection (124), wherein the reference electrode (134) is connected to the terminal for a supply voltage (Vs) by means of a third electrically conductive connection (136), wherein the pump electrode (142) is connected to the common terminal (COM) by means of the second electrically conductive connection (124), wherein the counter-electrode (144) is connected to the second separate terminal (P2) by means of a fourth electrically conductive connection (146), wherein a measurement resistor (160) is provided in the fourth electrically conductive connection (146), wherein a capacitance of the second pump cell (140) is changed by means of the control device (122) for a predetermined time in order to generate a measurement signal (180) at the measurement resistor (160), wherein a predetermined electric current (I_{CP}) is injected into the reference electrode (134) by the terminal for a supply voltage (Vs) by means of the third electrically conductive connection (136), wherein the predetermined electric current (I_{CP}) is changed for the predetermined time, wherein the fourth electrically conductive connection (146) is identified as intact if the measurement signal (180) has a change for the predetermined time and is identified as defective if the measurement signal (180) does not have any change for the predetermined time.

2. Method according to Claim 1, wherein the predetermined electric current (I_{CP}) is changed to a value of 0 µA for the predetermined time.

3. Method for operating a sensor (100) for detecting at least one portion of a measurement gas component with bound oxygen in a measurement gas, in particular in an exhaust gas of an internal combustion engine, comprising a sensor element (110), wherein the sensor element (110) a first pump cell (112) which has an outer pump electrode (114) and an inner pump electrode (116) and is present at a first cavity (126) connected to the measurement gas, a reference cell (130) which has a Nernst electrode (132) and a reference electrode (134) and is present at a reference gas space (138), and a second pump cell (140) which has a pump electrode (142) and a counter-electrode (144) and is present at a second cavity (145), wherein the counter-electrode (144) of the second pump cell (140) is in the reference gas space (138), wherein an electronic control device (122), which has at least a first separate terminal (P1) for the first pump cell (112), a second separate terminal (P2) for the second pump cell (140), a terminal for a supply voltage (Vs) and a common terminal (COM), is connected to the sensor element (110), wherein the outer pump electrode (114) is connected to the first separate terminal (P1) by means of a first by means of a first electrically conductive connection (120), wherein the inner pump electrode (116) is connected to the common terminal (COM) by means of a second electrically conductive connection (124), wherein the reference electrode (134) is connected to the terminal for a supply voltage (Vs) by means of a third electrically conductive connection (136), wherein the pump electrode (142) is connected to the common terminal (COM) by means of the second electrically conductive connection (124), wherein the counter-electrode (144) is connected to the second separate terminal (P2) by means of a fourth electrically conductive connection (146), wherein a measurement resistor (160) is provided in the fourth electrically conductive connection (146), wherein a capacitance of the second pump cell (140) is changed by means of the control device (122) for a predetermined time in order to generate a measurement signal (180) at the measurement resistor (160), wherein the sensor element (110) has a heating element (148), wherein the sensor element (110) is heated to a predetermined temperature by means of the heating element (148), wherein the predetermined temperature is changed for the predetermined time, wherein the fourth electrically conductive connection (146) is identified as intact if the measurement signal (180) has a change for the predetermined time and is identified as defective if the measurement signal (180) does not have any change for the predetermined time.

4. Method according to Claim 3, wherein the predetermined temperature is reduced for the predetermined time, wherein the fourth electrically conductive connection (146) is identified as intact if the measurement signal (180) has a reduction for the predetermined time and is identified as defective if the measurement signal (168) does not have any change for the predetermined time.

5. Method according to Claim 3, wherein the predetermined temperature is increased for the predetermined time, wherein the fourth electrically conductive connection (146) is identified as intact if the measurement signal (180) has an increase for the predetermined time and is identified as defective if the measurement signal (180) does not have any change for the predetermined time.

6. Method according to one of Claims 3 to 5, wherein the temperature of the sensor element (110) is determined by capturing an electrical internal resistance (R_{PVS}) of the sensor element (110).

7. Method according to one of Claims 1 to 6, wherein the measurement signal (180) is a voltage drop (U_{IP2}) across the measurement resistor (160).

8. Electronic control device having a first separate terminal (P1), a second separate terminal (P2), a terminal for a supply voltage (Vs) and a common terminal (COM) and having means which are suitable for carrying out the steps of the method according to one of the preceding claims.

9. Computer program comprising instructions which cause a control device according to Claim 8 to carry out the steps of the method according to one of Claims 1 to 7.

10. Electronic storage medium, on which a computer program according to the preceding claim is stored.

## Revendications

1. Procédé permettant de faire fonctionner un capteur (100) pour détecter au moins une fraction d'un composant de gaz de mesure avec de l'oxygène lié dans un gaz de mesure, en particulier dans des gaz d'échappement d'un moteur à combustion interne, comprenant un élément capteur (110), dans lequel l'élément capteur (110) une première cellule de pompage (112) qui présente une électrode de pompage extérieure (114) et une électrode de pompage intérieure (116) et qui est adjacente à une première cavité (126) qui communique avec le gaz de mesure, une cellule de référence (130) qui présente une électrode de Nernst (132) et une électrode de référence (134) et qui est adjacente à un compartiment de gaz de référence (138), et une deuxième cellule de pompage (140) qui présente une électrode de pompage (142) et une contre-électrode (144) et qui est adjacente à une deuxième cavité (145), dans lequel la contre-électrode (144) de la deuxième cellule de pompage (140) se trouve dans le compartiment de gaz de référence (138), dans lequel un appareil de commande électronique (122), qui dispose au moins d'une première borne séparée (P1) pour la première cellule de pompage (112), d'une deuxième borne séparée (P2) pour la deuxième cellule de pompage (140), d'une borne pour une tension d'alimentation (Vs) et d'une borne commune (COM), est relié à l'élément capteur (110), dans lequel l'électrode de pompage extérieure (114) est reliée à la première borne séparée (P1) au moyen d'une au moyen d'une première liaison (120) électriquement conductrice, dans lequel l'électrode de pompage intérieure (116) est reliée à la borne commune (COM) au moyen d'une deuxième liaison électriquement conductrice (124), dans lequel l'électrode de référence (134) est reliée à la borne pour une tension d'alimentation (Vs) au moyen d'une troisième liaison (136) électriquement conductrice, dans lequel l'électrode de pompage (142) est reliée à la borne commune (COM) au moyen d'une deuxième liaison (124) électriquement conductrice, dans lequel la contre-électrode (144) est reliée à la deuxième borne séparée (P2) au moyen d'une quatrième liaison (146) électriquement conductrice, dans lequel une résistance de mesure (160) est prévue dans la quatrième liaison (146) électriquement conductrice, dans lequel l'appareil de commande (122) permet de modifier une capacité de la deuxième cellule de pompage (140) pour générer un signal de mesure (180) au niveau de la résistance de mesure (160) pendant une durée prédéterminée, dans lequel un courant électrique (I_{CP}) prédéterminé est appliqué à l'électrode de référence (134) au moyen de la troisième liaison (136) électriquement conductrice à partir de la borne pour une tension d'alimentation (Vs), dans lequel le courant électrique (I_{CP}) prédéterminé est modifié pendant la durée prédéterminée, dans lequel la quatrième liaison (146) électriquement conductrice est identifiée comme intacte si le signal de mesure (180) présente une modification pendant la durée prédéterminée, et est identifiée comme défectueuse si le signal de mesure (180) ne présente aucune modification pendant la durée prédéterminée.

2. Procédé selon la revendication 1, dans lequel le courant électrique (I_{CP}) prédéterminé est modifié à une valeur de 0 µA pendant la durée prédéterminée.

3. Procédé permettant de faire fonctionner un capteur (100) pour détecter au moins une fraction d'un composant de gaz de mesure avec de l'oxygène lié dans un gaz de mesure, en particulier dans des gaz d'échappement d'un moteur à combustion interne, comprenant un élément capteur (110), dans lequel l'élément capteur (110) une première cellule de pompage (112) qui présente une électrode de pompage extérieure (114) et une électrode de pompage intérieure (116) et qui est adjacente à une première cavité (126) qui communique avec le gaz de mesure, une cellule de référence (130) qui présente une électrode de Nernst (132) et une électrode de référence (134) et qui est adjacente à un compartiment de gaz de référence (138), et une deuxième cellule de pompage (140) qui présente une électrode de pompage (142) et une contre-électrode (144) et qui est adjacente à une deuxième cavité (145), dans lequel la contre-électrode (144) de la deuxième cellule de pompage (140) se trouve dans le compartiment de gaz de référence (138), dans lequel un appareil de commande électronique (122), qui dispose au moins d'une première borne séparée (P1) pour la première cellule de pompage (112), d'une deuxième borne séparée (P2) pour la deuxième cellule de pompage (140), d'une borne pour une tension d'alimentation (Vs) et d'une borne commune (COM), est relié à l'élément capteur (110), dans lequel l'électrode de pompage extérieure (114) est reliée à la première borne séparée (P1) au moyen d'une première liaison (120) électriquement conductrice, dans lequel l'électrode de pompage intérieure (116) est reliée à la borne commune (COM) au moyen d'une deuxième liaison (124) électriquement conductrice, dans lequel l'électrode de référence (134) est reliée à la borne pour une tension d'alimentation (Vs) au moyen d'une troisième liaison (136) électriquement conductrice, dans lequel l'électrode de pompage (142) est reliée à la borne commune (COM) au moyen d'une deuxième liaison (124) électriquement conductrice, dans lequel la contre-électrode (144) est reliée à la deuxième borne séparée (P2) au moyen d'une quatrième liaison (146) électriquement conductrice, dans lequel une résistance de mesure (160) est prévue dans la quatrième liaison (146) électriquement conductrice, dans lequel l'appareil de commande (122) permet de modifier une capacité de la deuxième cellule de pompage (140) pour générer un signal de mesure (180) au niveau de la résistance de mesure (160) pendant une durée prédéterminée, dans lequel l'élément capteur (110) présente un élément chauffant (148), dans lequel l'élément capteur (110) est chauffé au moyen de l'élément chauffant (148) à une température prédéterminée, dans lequel la température prédéterminée est modifiée pendant la durée prédéterminée, dans lequel la quatrième liaison (146) électriquement conductrice est identifiée comme intacte si le signal de mesure (180) présente une modification pendant la durée prédéterminée, et est identifiée comme défectueuse si le signal de mesure (180) ne présente aucune modification pendant la durée prédéterminée.

4. Procédé selon la revendication 3, dans lequel la température prédéterminée est diminuée pendant la durée prédéterminée, dans lequel la quatrième liaison (146) électriquement conductrice est identifiée comme intacte si le signal de mesure (180) présente une diminution pendant la durée prédéterminée, et est identifiée comme défectueuse si le signal de mesure (168) ne présente aucune modification pendant la durée prédéterminée.

5. Procédé selon la revendication 3, dans lequel la température prédéterminée est augmentée pendant la durée prédéterminée, dans lequel la quatrième liaison (146) électriquement conductrice est identifiée comme intacte si le signal de mesure (180) présente une augmentation pendant la durée prédéterminée, et est identifiée comme défectueuse si le signal de mesure (180) ne présente aucune modification pendant la durée prédéterminée.

6. Procédé selon l'une quelconque des revendications 3 à 5, dans lequel la température de l'élément capteur (110) est déterminée par la détection d'une résistance électrique intérieure (R_{PVS}) de l'élément capteur (110).

7. Procédé selon l'une quelconque des revendications 1 à 6, dans lequel le signal de mesure (180) est une chute de tension (U_{IP2}) aux bornes de la résistance de mesure (160) .

8. Appareil de commande électronique qui dispose d'une première borne séparée (P1), d'une deuxième borne séparée (P2), d'une borne pour une tension d'alimentation (Vs) et d'une borne commune (COM), et de moyens qui sont adaptés pour exécuter les étapes du procédé selon l'une quelconque des revendications précédentes.

9. Programme informatique, comprenant des instructions qui font qu'un appareil de commande selon la revendication 8 exécute les étapes du procédé selon l'une quelconque des revendications 1 à 7.

10. Support de stockage électronique sur lequel est stocké un programme informatique selon la revendication précédente.
